Europäisches Patentamt

European Patent Office

Office européen des brevets

(19)

(11) EP 0 969 793 B1

(12) EUROPÄISCHE PATENTSCHRIFT

(45) Veröffentlichungstag und Bekanntmachung des Hinweises auf die Patenterteilung:
**30.06.2004 Patentblatt 2004/27**

(51) Int Cl.[7]: **A61K 7/00**, A45D 7/04, A61K 7/06

(21) Anmeldenummer: **98958886.8**

(22) Anmeldetag: **31.10.1998**

(86) Internationale Anmeldenummer:
**PCT/EP1998/006905**

(87) Internationale Veröffentlichungsnummer:
**WO 1999/029282 (17.06.1999 Gazette 1999/24)**

(54) **Verwendung einer Folie zum Einwickeln der Haarspitzen vor der Dauerwellbehandlung und Folie.**

Use of a foil for wrapping hair ends before permanent wave treatment and said foils.

Utilisation d'une feuille pour envelopper les pointes de cheveux avant la déformation permanente et ces feuilles.

(84) Benannte Vertragsstaaten:
**DE ES FR GB IT**

(30) Priorität: **05.12.1997 DE 19753962**

(43) Veröffentlichungstag der Anmeldung:
**12.01.2000 Patentblatt 2000/02**

(73) Patentinhaber: **Wella Aktiengesellschaft 64274 Darmstadt (DE)**

(72) Erfinder:
• **SCHONERT, Dieter D-64354 Reinheim (DE)**
• **MARESCH, Gerhard D-64295 Darmstadt (DE)**
• **MECKS, Gernot D-64739 Höchst (DE)**

(56) Entgegenhaltungen:
**EP-A- 0 328 355        WO-A-97/46205**
**DE-A- 1 492 007        US-A- 4 601 299**
**US-A- 5 837 697**

EP 0 969 793 B1

**Beschreibung**

Die Erfindung betrifft die Verwendung einer imprägnierten Folie zum Einwickeln der Haarspitzen (Spitzenpapier) vor der Dauerwellbehandlung in Friseurbetrieben sowie eine neue Folie.

Zur Herstellung einer permanenten Haarverformung am menschlichen Haar, sind neben der Anwendung eines Reduktions- und Oxidationsmittels, Wickelkörper erforderlich. Das Haar wird auf seiner Längsachse von den Haarspitzen zum kopfhautnahen Ansatzbereich aufgerollt. Durch die mehrfache Umdrehung der Haare um den Wickler wird zwangsläufig die Haarspitze kleinlockiger als das Haar im Ansatzbereich. Die kleinlockige Spitzenumformung ist jedoch bei der Frisurenerstellung hinderlich und unerwünscht. Erschwerend kommt hinzu, daß das Haar im ungeschädigten (nativen) Zustand des Ansatzbereiches noch eine geschlossenere Cuticula aufweist. Dadurch ist der Ansatzbereich resistenter im Vergleich zu den um Monate älteren und durch den Einfluß des Kämmens, Waschens, Bleichens, Färbens oder Wellens und von Umwelteinflüssen zunehmend spröderen und durchlässigeren Spitzenbereich.

Die zuvor erwähnten Nachteile bezüglich der Haarspitzen führen zu einer Verschlechterung der Struktur und der Frisierbarkeit.

Wiederholt wurde der Versuch unternommen, mittels Dauerwellvorbehandlungsmittel (z. B. WO-A 97/09028) oder mit Säuren (z. B. DE-A 33 11 292 und DE-A 1 492 007) oder Ölen (z. B. DE-A 42 36 726) getränktem Spitzenpapier ein gleichmäßiges Wellergebnis und einen Spitzenschutz/Strukturausgleich zu erreichen. Dauerwellvorbehandlungsmittel werden als Flüssigkeit oder als Gel dargeboten. Die Praxis zeigt, daß bei beiden Konsistenzen Applikations- bzw. Dosierprobleme nicht zu verhindern sind. Ein nur mit Säuren oder nur mit Öl getränktes Spitzenpapier ist in seiner Wirkung bezüglich des Well- und Strukturausgleichs zu wirkungsschwach. In der WO-A- 97/46205 Beispiel 2 ist ein im wasserfreies antivirales Tuch zur Desinfektion der Haut beschrieben, welches mit einer Lotion, enthaltend 25 Gew. % Petrolatum, 20 Gew.% Ceteareth-10 und 15 Gew.% eines Gemisches aus organischen Säuren besprüht wurde.

Aufgabe der vorliegenden Erfindung ist es, den oben genannten Überkrausungseffekt durch eine neue Verwendung sowie eine neue Folie besser zu vermeiden, so daß während der Einwirkungszeit des Wellpräparates in besonderer Weise Längen und Spitzen gegenüber dem herkömmlichen Verfahren geschont werden und so die bekannten Nachteile vermieden werden. Damit sollte die Haarstruktur weniger geschädigt werden und die Längen und Spitzen einen vergleichbaren Wellenradius, wie der des kopfhautnahen Ansatzbereiches, trotz engerer Windungen, erhalten.

Überraschend wurde nun gefunden, daß durch die Verwendung einer Folie zum Einwickeln der Haarspitzen, welche mit einer wäßrigen Zubereitung, enthaltend

   a) 20 bis 70 Gew.% einer lipophilen Substanz,
   b) 1 bis 25 Gew.% eines Emulgators und
   c) 0,5 bis 15 Gew.% einer organischen Säure,

getränkt ist, für ein Verfahren zur Dauerwellung von Haaren es überraschenderweise gelingt, das unerwünschte und zu kleinlockige Wellergebnis in den Haarspitzen zu verhindern. Gleichzeitig wird der empfindliche Teil des Haares durch die natürlichen Öle geschützt und durch die Säuren einer übermäßigen Quellung entgegen gewirkt.

Ein weiterer Erfindungsgegenstand ist eine Folie nach Anspruch 10.

Folien wie Spitzenpapiere für die Dauerwelle sind an sich bekannt, sie bestehen üblicherweise aus naßfestem Papier, z. B. Langfaser-, Seiden- oder Japanpapier. Die Folie kann jedoch auch aus einem anderen saugfähigen Material wie z. B. Vliesstoff, Baumwollgewebe oder Gewebegemischen von Kunststofffasem mit Naturfasern bestehen.

Die lipophile Substanz ist in der wäßrigen Zubereitung bevorzugt in einer Menge von 40 bis 60 Gew.% enthalten. Sie ist bevorzugt ein natürliches Öl oder Wachs, ausgewählt aus physiologisch verträglichen, insbesondere ungesättigten, natürlichen hydrophoben Ölen oder Wachsen. Besonders bevorzugt ist sie ein pflanzliches Öl wie z. B. Jojobaöl, Avocadoöl, Sonnenblumenöl, Weizenkeimöl, Pfirsichkemöl, Nerzöl, Ricinusöl, Sesamöl, Erdnußöl, Rüböl, Baumwollsaatöl oder Sojaöl. Geeignet sind auch physiologisch verträgliche natürliche Öle oder Wachse wie Montanwachs, Erdwachs, Vaseline und Paraffin oder synthetische Öle, z. B. Silikonöle.

Der Emulgator ist in der wäßrigen Zubereitung bevorzugt in einer Menge von 2 bis 15 Gew.% und ganz besonders bevorzugt 3 bis 10 Gew.%, enthalten. Er ist vorzugsweise ausgewählt aus mit 2 bis 200 Ethylenoxidgruppen ethoxylierten Verbindung aus der Gruppe der Fettsäuren, Fettsäureamide, Fettsäureamine oder Fettalkohole mit jeweils 6 bis 30 Kohlenstoffatomen sowie der Fettsäureester mit jeweils 6 bis 30 Kohlenstoffatomen im Fettsäurerest. Besonders geeignete Emulgatoren sind z. B. mit 7 Ethylenoxidgruppen oxethyliertes, hydriertes Rizinusöl (z. B. Arlacel® 989 der Firma ICI) und mit 40 Ethylenoxidgruppen oxethyliertes hydriertes Rizinusöl (z. B. Cremophor® RH40 der Firma BASF).

Die organische Säure ist in der wäßrigen Zubereitung bevorzugt in einer Menge von 1 bis 7 Gew.%, besonders bevorzugt 2 bis 6 Gew.%, enthalten. Sie ist bevorzugt eine Hydroxycarbonsäure oder eine Aldehyd- oder Ketocarbonsäure. Beispiele für geeignete Säuren sind Glyoxylsäure, Zitronensäure, Ascorbinsäure, Milchsäure, Weinsäure, Essigsäure, Aconitsäure, Acetylendicarbonsäure, Ethylendicarbonsäure, Athylenmaleinsäure, $\alpha$-Ethylcrotonsäure, i-Amylmaleinsäure, Angelicasäure, n-Butylfumarsäure, n- oder i-Butytmaleinsäure, Citraconsäure, Crotonsäure, Fumarsäure, trans-Glutaconsäure, Isopropyimaleinsäure, Itaconsäure, Maleinsäure, Mesaconsäure, $\alpha$-Methylitaconsäure, cis-p-Methylglutaconsäure, trans-$\alpha$-Methylglutaconsäure, Propiolsäure und Zimtsäure,

Die wäßrige Zubereitung enthält vorzugsweise 30 bis 60 Gew.% Wasser; sie kann darüber hinaus ggf. weitere Zusätze wie Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Parfümöle und Farbstoffe in einer Menge von jeweils 0,01 bis 1 Gewichtsprozent, die Puffersubstanzen in einer Menge von insgesamt 0,1 bis 10 Gewichtsprozent, Stabilisatoren, sowie haarkonditionierende und haarpflegende Bestandteile in einer Menge von jeweils 0,1 bis 5 Gewichtsprozent..

Vergleichsversuche an Probanden bei dem das Spitzenpapier nur mit Ölen oder nur mit Säuren getränkt wurde, führten zu deutlich schwächeren und unbefriedigenden Ergebnissen. Sie bestätigen somit sehr eindrucksvoll die oben aufgeführte Wirkung.

## BEISPIELE

### Beispiel 1

Ein Gewichtsteil einer 5 %igen wäßrigen Lösung von Glyoxylsäure wird mit einem Gewichtsteil einer Mischung aus 84 Gew.% Jojobaöl und 16 Gew.% eines hydrierten polyoxethylierten Rizinusöls mit 40 Ethylenoxidgruppen (Cremophor® RH 40 der Firma BASF) innig zu einer Emulsion vermischt.

Es wird eine Emulsion der nachfolgenden Zusammensetzung erhalten:

| | |
|---|---|
| 2,5 Gew.% | Glyoxylsäure |
| 44,0 Gew.% | Jojobaöl |
| 8,0 Gew.% | hydriertes polyoxethylierten Rizinusöls mit 40 Ethylenoxid-gruppen (Cremophor® RH 40 der Firma BASF) |
| 45.5 Gew.% | Wasser |
| 100,0 Gew.% | |

Mit dieser Emulsion wird ein herkömmliches Dauerwellspitzenpapier intensiv getränkt, die überschüssige Emulsion abgestreift sowie die verbliebene angetrocknet. Durch die Anwendung des erhaltenen, bedingt "feuchten" Dauerwellspitzenpapiers wird eine extreme Schonung der Haarspitzen erreicht, dessen Folge ein lang anhaftender, sanfter Wellenbogen des Haares ist.

### Beispiel 2

Ein Gewichtsteil einer 5 Gew.%igen wäßrigen Lösung von Milchsäure wird mit einem Gewichtsteil einer Mischung aus 90 Gew.% Avocadoöl und 10 Gew.% eines hydrierten polyoxethylierten Rizinusöls mit 40 Ethylenoxidgruppen (Cremophor® RH 40 der Firma BASF) innig zu einer Emulsion vermischt.

Es wird eine Emulsion der nachfolgenden Zusammensetzung erhalten:

| | |
|---|---|
| 2,5 Gew.% | Milchsäure |
| 45,0 Gew.% | Avocadoöl |
| 5,0 Gew.% | hydriertes polyoxethylierten Rizinusöls mit 40 Ethylenoxid-gruppen (Cremophor® RH 40 der Firma BASF) |
| 47,5 Gew.% | Wasser |
| 100,0 Gew.% | |

Mit dieser Emulsion wird ein herkömmliches Dauerwellspitzenpapier intensiv getränkt, die überschüssige Emulsion abgestreift sowie die verbliebene angetrocknet. Durch die Anwendung des erhaltenen, bedingt "feuchten" Dauerwellspitzenpapiers wird eine extreme Schonung der Haarspitzen erreicht, dessen Folge ein lang anhaftender, sanfter Wellenbogen des Haares ist.

**Beispiel 3**

Ein Gewichtsteil einer 3 Gew.%igen wäßrigen Zitronensäurelösung wird mit einem Gewichtsteil einer Mischung aus

| 33,0 Gew.% | buxus chinensis (Jojobaöl) |
|---|---|
| 32,0 Gew.% | helianthus annuus (Sonnenblumenöl) |
| 31,0 Gew.% | persea gratissima (Avocadoöl) |
| 2,0 Gew.% | mit 40 Ethylenoxidgruppen oxyethyliertes hydriertes Rizinusöl (Cremophor® RH40 der Firma BASF) |
| 1,0 Gew.% | mit 7 Ethylenoxidgruppen oxyethyliertes, hydriertes Rizinusöl (Arlacel® 989 der Firma ICI) |
| 0,1 Gew.% | Antioxidans |
| 0,9 Gew.% | Wasser |
| 100,0 Gew.% | |

zu einer Emulsion vermischt.

Es wird eine Emulsion der nachfolgenden Zusammensetzung erhalten:

| 16,50 Gew.% | buxus chinensis (Jojobaöl) |
|---|---|
| 16,00 Gew.% | helianthus annuus (Sonnenblumenöl) |
| 15,50 Gew.% | persea gratissima (Avocadoöl) |
| 1,00 Gew.% | mit 40 Ethylenoxidgruppen oxyethyliertes hydriertes Rizinusöl (Cremophor® RH40 der Firma BASF) |
| 0,50 Gew.% | mit 7. Ethylenoxidgruppen oxyethyliertes, hydriertes Rizinusöl (Arlacel® 989 der Firma ICI) |
| 1,50 Gew.% | Zitronensäure |
| 0,05 Gew.% | Antioxidans |
| 48,95 Gew.% | Wasser |
| 100,00 Gew.% | |

Mit dieser Emulsion wird ein herkömmliches Dauerwellspitzenpapier intensiv getränkt, die überschüssige Emulsion abgestreift sowie die verbliebene angetrocknet. Durch die Anwendung des erhaltenen, bedingt "feuchten" Dauerwellspitzenpapiers wird eine extreme Schonung der Haarspitzen erreicht, dessen Folge ein langanhaltender, sanfter Wellenbogen des Haares ist.

**Patentansprüche**

1. Verwendung einer Folie zum Einwickeln der Haarspitzen, **dadurch gekennzeichnet, daß** sie mit einer wäßrigen Zubereitung, enthaltend

   a) 20 bis 70 Gew.% einer lipophilen Substanz,
   b) 1 bis 25 Gew.% eines Emulgators und
   c) 0,5 bis 15 Gew.% einer organischen Säure,

   getränkt für ein Verfahren zur Dauerwellung von Haaren.

2. Verwendung nach Anspruch 1, **dadurch gekennzeichnet, daß** die lipophile Substanz in einer Menge von 40 bis 60 Gew.% enthalten ist.

3. Verwendung nach einem der Ansprüche 1 oder 2, **dadurch gekennzeichnet, daß** die lipophile Substanz ein physiologisch verträgliches ungesättigtes natürliches hydrophobes Öl oder Wachs ist.

4. Verwendung nach einem der Ansprüche 1 bis 3, **dadurch gekennzeichnet, daß** die lipophile Substanz ein Öl oder Wachs, ausgewählt aus den physiologisch verträglichen natürlichen hydrophoben Ölen und Wachsen Jojobaöl, Avocadoöl, Sonnenblumenöl, Weizenkeimöl, Pfirsichkernöl, Nerzöl, Sojaöl, Ricinusöl, Sesamöl, Erdnußöl, Rüböl, Baumwollsaatöl, Montanwachs, Erdwachs, Vaseline und Paraffin sowie dem physiologisch verträglichen synthetischen Öl Silikonöl ist.

5. Verwendung nach einem der Ansprüche **1** bis **4, dadurch gekennzeichnet, daß** der Emulgator in der wäßrigen Zubereitung bevorzugt in einer Menge von 2 bis 15 Gew.% enthalten ist.

6. Verwendung nach einem der Ansprüche 1 bis 5, **dadurch gekennzeichnet, daß** der Emulgator ausgewählt ist aus einer mit 2 bis 200 Ethylenoxidgruppen ethoxylierten Verbindung aus der Gruppe der Fettsäuren, Fettsäureamide, Fettsäureamine oder Fettalkohole mit jeweils 6 bis 30 Kohlenstoffatomen sowie der Fettsäureester mit jeweils 6 bis 30 Kohlenstoffatomen im Fettsäurerest.

7. Verwendung nach Anspruch 6, **dadurch gekennzeichnet, daß** der Emulgator ausgewählt ist aus mit 7 Ethylenoxidgruppen oxethyliertem, hydrierten Rizinusöl und mit 40 Ethylenoxidgruppen oxethyliertem hydrierten Rizinusöl.

8. Verwendung nach einem der Ansprüche 1 bis 7 **dadurch gekennzeichnet, daß** die organische Säure in der wäßrigen Zubereitung in einer Menge von 1 bis 7 Gew. enthalten ist.

9. Verwendung nach Anspruch 8, **dadurch gekennzeichnet, da**ß die organische Säure ausgewählt ist aus Glyoxylsäure, Zitronensäure, Ascorbinsäure, Milchsäure, Weinsäure, Essigsäure, Aconitsäure, Acetylendicarbonsäure, Ethylendicarbonsäure, Athylenmaleinsäure $\alpha$-Ethylcrotonsäure, i-Amylmaleinsäure, Angelicasäure, n-Butylfumarsäure, n- oder i-Butytmaleinsäure, Citraconsäure, Crotonsäure, Fumarsäure, trans-Glutaconsäure, Isopropylmaleinsäure, Itaconsäure, Maleinsäure, Mesaconsäure, $\alpha$-Methylitaconsäure, cis-p-Methylglutaconsäure, trans-$\alpha$-Methylglutaconsäure, Propiolsäure und Zimtsäure.

10. Folie zum Einwickeln der Haarspitzen, **dadurch gekennzeichnet, dass** sie mit einer wässrigen Zubereitung, enthaltend

   a) 40 bis 60 Gew.% einer lipophilen Substanz,
   b) 1 bis 25 Gew.% eines Emulgators und
   c) 0,5 bis 15 Gew.% einer organischen Säure und
   d) 30 bis 60 Gew. % Wasser

getränkt ist.


## Claims

1. Use of a film for wrapping hair ends, **characterized in that** it is impregnated with an aqueous preparation comprising

   a) 20 to 70% by weight of a lipophilic substance,
   b) 1 to 25% by weight of an emulsifier and
   c) 0.5 to 15% by weight of an organic acid,

for a method for the permanent waving of hair.

2. Use according to Claim 1, **characterized in that** the lipophilic substance is present in an amount of from 40 to 60% by weight.

3. Use according to one of Claims 1 or 2, **characterized in that** the lipophilic substance is a physiologically compatible unsaturated natural hydrophobic oil or wax.

4. Use according to one of Claims 1 to 3, **characterized in that** the lipophilic substance is an oil or wax chosen from the physiologically compatible natural hydrophobic oils and waxes jojoba oil, avocado oil, sunflower oil, wheatgerm oil, peach kernel oil, mink oil, soybean oil, castor oil, sesame oil, peanut oil, rapeseed oil, cottonwool seed oil, montan wax, earth wax, petroleum jelly and paraffin, and the physiologically compatible synthetic oil is silicone oil.

5. Use according to one of Claims 1 to 4, **characterized in that** the emulsifier is present in the aqueous preparation preferably in an amount of from 2 to 15% by weight.

6. The use as claimed in one of Claims 1 to 5, **characterized in that** the emulsifier is chosen from a compound, ethoxylated with 2 to 200 ethylene oxide groups, from the group of fatty acids, fatty acid amides, fatty acid amines or fatty alcohols having in each case 6 to 30 carbon atoms, and of fatty acid esters having in each case 6 to 30 carbon atoms in the fatty acid radical.

7. Use according to Claim 6, **characterized in that** the emulsifier is chosen from hydrogenated castor oil oxyethylated with 7 ethylene oxide groups and hydrogenated castor oil oxyethylated with 40 ethylene oxide groups.

8. Use according to one of Claims 1 to 7, **characterized in that** the organic acid is present in the aqueous preparation in an amount of from 1 to 7% by weight.

9. Use according to Claim 8, **characterized in that** the organic acid is chosen from glyoxylic acid, citric acid, ascorbic acid, lactic acid, tartaric acid, acetic acid, aconitic acid, acetylenedicarboxylic acid, ethylenedicarboxylic acid, ethylenemaleic acid, $\alpha$-ethylcrotonic acid, i-amylmaleic acid, angelic acid, n-butylfumaric acid, n- or i-butylmaleic acid, citraconic acid, crotonic acid, fumaric acid, trans-glutaconic acid, isopropylmaleic acid, itaconic acid, maleic acid, mesaconic acid, $\alpha$-methylitaconic acid, cis-p-methylglutaconic acid, trans-$\alpha$-methylglutaconic acid, propiolic acid and cinnamic acid.

10. Film for wrapping hair ends, **characterized in that** it is impregnated with an aqueous preparation comprising

    a) 40 to 60% by weight of a lipophilic substance,
    b) 1 to 25% by weight of an emulsifier and
    c) 0.5 to 15% by weight of an organic acid and
    d) 30 to 60% by weight of water.

**Revendications**

1. Utilisation d'une pellicule pour l'enroulement des pointes des cheveux, **caractérisée en ce qu'**elle est imprégnée avec une préparation aqueuse contenant

    a) 20 à 70 % en poids d'une substance lipophile,
    b) 1 à 25 % en poids d'un émulsifiant et
    c) 0,5 à 15 % en poids d'un acide organique,

    pour un procédé destiné à l'ondulation permanente des cheveux.

2. Utilisation selon la revendication 1, **caractérisée en ce que** la substance lipophile est contenue en une quantité de 40 à 60 % en poids.

3. Utilisation selon la revendication 1 ou 2, **caractérisée en ce que** la substance lipophile est une huile ou cire naturelle insaturée hydrophobe physiologiquement acceptable.

4. Utilisation selon l'une quelconque des revendications 1 à 3, **caractérisée en ce que** la substance lipophile est une huile ou cire choisie parmi les huiles et cires naturelles hydrophobes physiologiquement acceptables huile de jojoba, huile d'avocat, huile de tournesol, huile de germes de blé, huile de noyaux de pêche, huile de vison, huile de soja, huile de ricin, huile de sésame, huile d'arachide, huile de betterave, huile de coton, cire de lignite, cire minérale, vaseline et paraffine, ainsi l'huile synthétique physiologiquement acceptable huile de silicone.

5. Utilisation selon l'une quelconque des revendications 1 à 4, **caractérisée en ce que** l'émulsifiant est contenu dans la préparation aqueuse de préférence en une quantité de 2 à 15 % en poids.

6. Utilisation selon l'une quelconque des revendications 1 à 5, **caractérisée en ce que** l'émulsifiant est choisi parmi des composés éthoxylés avec 2 à 200 groupes oxyde d'éthylène choisis dans le groupe des acides gras, amides d'acides gras, amines d'acides gras et alcools gras ayant chacun de 6 à 30 atomes de carbone, ainsi que des esters d'acides gras ayant chacun de 6 à 30 atomes de carbone dans le reste acide gras.

7.  Utilisation selon la revendication 6, **caractérisée en ce que** l'émulsifiant est choisi parmi l'huile de ricin hydrogénée, oxyéthylée avec 7 groupes oxyde d'éthylène et l'huile de ricin hydrogénée, oxyéthylée avec 40 groupes oxyde d'éthylène.

8.  Utilisation selon l'une quelconque des revendications 1 à 7, **caractérisée en ce que** l'acide organique est contenu dans la préparation aqueuse en une quantité de 1 à 7 % en poids.

9.  Utilisation selon la revendication 8, **caractérisée en ce que** l'acide organique est choisi parmi l'acide glyoxylique, l'acide citrique, l'acide ascorbique, l'acide lactique, l'acide tartrique, l'acide acétique, l'acide aconitique, l'acide acétylènedicarboxylique, l'acide éthylènedicarboxylique, l'acide éthylènemaléique, l'acide $\alpha$-éthylcrotonique, l'acide isoamylmaléique, l'acide angélique, l'acide n-butylfumarique, l'acide n- ou isobutylmaléique, l'acide citraconique, l'acide crotonique, l'acide fumarique, l'acide *trans*-glutaconique, l'acide isopropylmaléique, l'acide itaconique, l'acide maléique, l'acide mésaconique, l'acide $\alpha$-méthylitaconique, l'acide cis-p-méthylglutaconique, l'acide trans-$\alpha$-méthylglutaconique, l'acide propiolique et l'acide cinnamique.

10. Pellicule pour l'enroulement des pointes des cheveux, **caractérisée en ce qu'**elle est imprégnée avec une préparation aqueuse contenant

    a) 40 à 60 % en poids d'une substance lipophile,
    b) 1 à 25 % en poids d'un émulsifiant et
    c) 0,5 à 15 % en poids d'un acide organique et
    d) 30 à 60 % en poids d'eau.